# EUROPEAN PATENT APPLICATION

(11) **EP 2 774 506 A2**
(43) Date of publication of application: **10.09.2014**
(21) Application number: 13180263.9
(22) Date of filing: 13.08.2013
(51) Int. Cl.: A45D 29/00, A61Q 3/02, A61K 8/04

(54) **Nail sticker for coating UV gel layer and application method thereof**

(30) Priority: 05.03.2013 KR 20130023092
(71) Applicant: JC Korea Corp., Gyeonggi-do (KR)
(72) Inventor: Kim, Dong Sung, Gyeonggi-do (KR); KIim Hyun Surk, Gyeonggi-do (KR); Choi, Kyung Sik, Gyeonggi-go (KR); Park, Ju Young, Gyeonggi-do (KR); Choi, Jeong Rim, Gyeonggi-do (KR); Kim, Bo Mi, Seoul (KR)
(74) Representative: Gassner, Wolfgang

(57) **Abstract**

The present disclosure relates to a nail sticker including a UV gel layer and a method for preparing same. In accordance with the embodiments of the present disclosure, the need of soaking a nail in a harmful solution for a long time for removal of a UV gel may be avoided while maintaining the advantages of the UV gel. Also, the problems that the opposite end of the cuticle is chipped or the end of the cuticle is peeled may be solved while maintaining the advantages of the UV gel.

## Description

### TECHNICAL FIELD

The present disclosure relates to a nail sticker for coating a UV gel layer and an application method thereof.

### BACKGROUND

As a way of beautifying human body, the beauty industry is developing fast and being specialized. The beauty industry began to be popularized in the 20th century with rapid market growth. In particular, nail art is developing fast recently as a way of beautification without regard to age or sex.

Nail art was originally a field of beauty art and the length, shape or color of nail has been expressed reflecting the cultural value of the age.

Various materials have been used in the nail art. Recently, ultraviolet (UV) gel is in the limelight. The UV gel hardens under UV light and has many advantages. It does not break easily because it is flexible and soft, does not suffer discoloration even after exposure to sunlight and exhibits excellent gloss. The UV gel is obtained by applying a composition for forming a UV gel layer on a natural nail and curing by irradiating UV. Since the UV gel can provide a glossy and shiny effect to the natural nail, it is widely used.

Despite these advantages, if the UV gel layer is formed directly on the natural nail, the finger has to be soaked in a harmful solvent such as acetone for a long time to remove it. This process may be detrimental to the skin and the nail and the natural nail may be severely damaged after the UV gel layer is removed. In addition, there are problems that the opposite end of the cuticle is chipped or the end of the cuticle is peeled.

### SUMMARY

The present disclosure is directed to overcoming the disadvantage of a UV gel while maintaining its advantages by attaching a nail sticker on a natural nail and then coating a UV gel layer thereon.

In accordance with the embodiments of the present disclosure, by attaching a nail sticker on a natural nail and then coating a UV gel layer thereon, the decorating effect of the UV gel layer can be maintained while allowing easy removal of the UV gel without using a harmful organic solvent.

In particular, the present disclosure is directed to providing a nail sticker which avoids the need of soaking a nail in a harmful solution for a long time for removal of a UV gel while maintaining the advantages of the UV gel. The present disclosure is also directed to providing a nail sticker which can solve the problems that the opposite end of the cuticle is chipped or the end of the cuticle is peeled maintaining the advantages of the UV gel.

If the existing nail sticker is attached on a natural nail and then a UV gel layer is coated thereon, adhesion between the nail sticker and the UV gel layer may be insufficient. Thus, the present disclosure is also directed to providing a nail sticker for coating a UV gel layer wherein, after the nail sticker is attached on a natural nail and then a UV gel layer is coated thereon, the UV gel layer can be stably attached without interlayer delamination for at least 3 days on average.

In a general aspect, there is provided a nail sticker for coating a UV gel layer, wherein a top surface of the nail sticker is embossed.

In another general aspect, there is provided a nail sticker for coating a UV gel layer, which has a print layer formed on a top surface of the nail sticker.

In another general aspect, there is provided a nail sticker for coating a UV gel layer, which has a UV-cured coating layer formed on a top surface of the nail sticker.

In another general aspect, there is provided a nail sticker for coating a UV gel layer, which has a top primer layer formed on a top surface of the nail sticker.

In another general aspect, there is provided a nail sticker for coating a UV gel layer, which has a laminated film layer attached on a top surface of the nail sticker.

In another general aspect, there is provided a UV gel layer-coated nail sticker, which has a UV gel layer formed on a nail sticker.

In another general aspect, there is provided a nail sticker kit for coating a UV gel layer including (i) a nail sticker and (ii) a composition for forming a UV-cured coating layer.

In another general aspect, there is provided a nail care method using a UV gel layer-coated nail sticker, including: (S1) attaching a nail sticker on a natural nail; (S2) coating a composition for forming a UV base gel layer on the nail sticker; (S3) forming a UV base gel layer by exposing the coated composition for forming a UV base gel layer to UV light; (S4) coating a composition for forming a UV top gel layer on the UV base gel layer; and (S5) forming a UV top gel layer by exposing the coated composition for forming a UV top gel layer to UV light.

### BRIEF DESCRIPTION OF THE DRAWINGS

The above and other objects, features and advantages of the present disclosure will become apparent from the following description of certain exemplary embodiments given in conjunction with the accompanying drawings, in which:

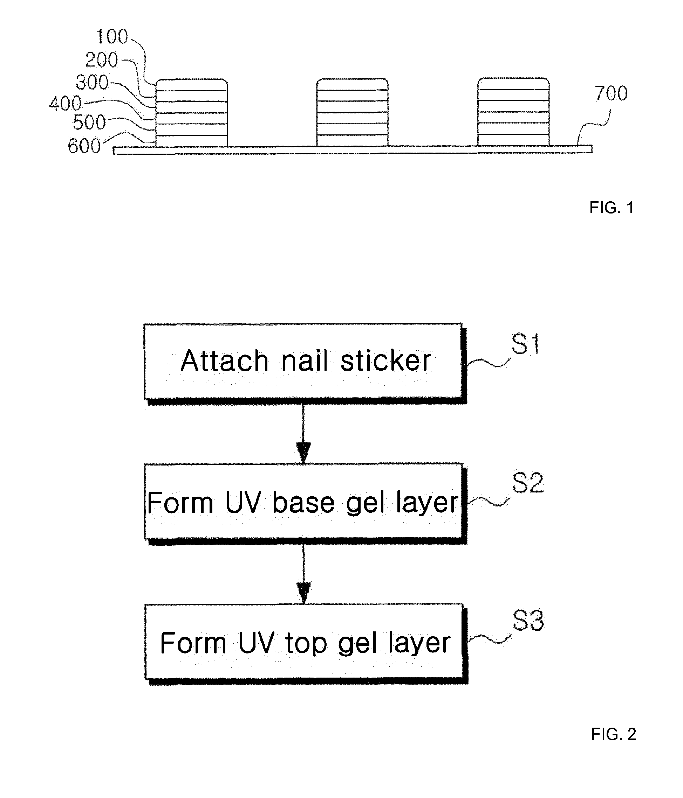

FIG. 1 is a cross-sectional view of a nail sticker according to an exemplary embodiment of the present disclosure, but the scope of the present disclosure is not limited by FIG. 1;
FIG. 2 describes a method for preparing a nail sticker according to an exemplary embodiment of the present disclosure;
FIG. 3 shows a nail sticker for coating a UV gel layer having an embossed surface according to an exemplary embodiment of the present disclosure; and
FIG. 4 shows a nail sticker for coating a UV gel layer having a print layer according to another exemplary embodiment of the present disclosure.

### [Detailed Description of Main Elements]

| | |
|---|---|
| 100: UV top gel layer | 200: UV base gel layer |
| 300: top coating layer | 400: ink layer |
| 500: substrate | 600: adhesion layer |
| 700: release paper | |

### DETAILED DESCRIPTION OF EMBODIMENTS

The advantages, features and aspects of the present disclosure will become apparent from the following description of the embodiments with reference to the accompanying drawings, which is set forth hereinafter.

In order to provide a nail sticker for coating a UV gel layer wherein the UV gel layer can be stably attached without interlayer delamination, the present disclosure suggests (i) physical roughness treatment (embossing or patterning) of surface, (ii) surface treatment through printing, (iii) introduction of a UV-cured top coating layer, (iv) introduction of a top primer layer and (v) introduction of a laminated film layer for all or part of the surface of the nail sticker for coating a UV gel layer.

Combinations of two or more of the four methods are also included in the scope of the present disclosure.

The print layer, the UV-cured coating layer, the primer layer and the laminated film layer serve to protect the natural nail and the nail sticker and to enhance adhesion between the nail sticker and the UV gel layer.

Not just the nail sticker for coating a UV gel layer, a nail sticker having a UV gel layer coated on the nail sticker for coating a UV gel layer according to the present disclosure is also included in the scope of the present disclosure. The UV gel layer may consist of either a single layer or a plurality of layers such as a base gel layer and a top gel layer

In a representative exemplary embodiment, fine roughness may be provided to a top surface of the nail sticker.

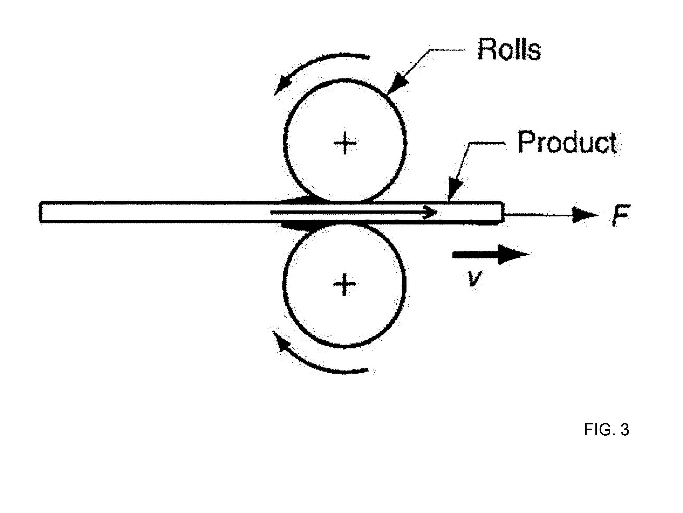

In an exemplary embodiment, the top surface of the nail sticker may be embossed by passing the nail sticker between rolls, as shown in FIG. 3. As a result, adhesion between the nail sticker and the UV gel layer is enhanced because surface area and contact area with the UV gel layer are increased.

When the resulting embossing pattern has a surface roughness satisfying the conditions described below, fine wrinkles are formed on the surface of the nail sticker. Since the fine wrinkles firmly hold the UV gel layer, peeling of the UV gel layer is greatly inhibited particularly at both ends.

An embossing pattern may be formed on the surface of the roll. The shape, depth, size, etc. of the embossing pattern formed on the nail sticker may be controlled by varying the embossing pattern on the roll surface, pressure between the roles, temperature, and the materials or physical properties of the nail sticker.

In this manner, a regular pattern may be formed. However, if the same effect can be achieved by forming an irregular pattern by another method, it will be also included in the scope of the present disclosure.

In particular, when the surface roughness according to JIS B 0601-1982 satisfies 0.04a ≤ Rₐ ≤ 100a and 1.6z ≤ R_{z} ≤ 400z, the UV gel layer can be stably attached without interlayer delamination for at least 3 days on average.

In addition, when the surface roughness satisfies 1a ≤ Rₐ ≤ 50a and 5z ≤ R_{z} ≤ 200z, not only the adhesion between the nail sticker and the UV gel layer is ensured as descried above but also the problem that the end of the cuticle is peeled can be solved.

Particularly, when the surface roughness satisfies 5a ≤ Rₐ ≤ 10a and 10z ≤ R_{z} ≤ 100z, the problem that the opposite end of the cuticle is chipped can be further solved in addition to the enhancement of adhesion between the nail sticker and the UV gel layer and the prevention of peeling at the end of the cuticle. That is to say, since the fine wrinkles formed on the surface of the nail sticker by embossing firmly hold the UV gel layer when the surface roughness satisfies the above-described condition, the problem that the both ends of the UV gel layer are peeled can be completely prevented.

In another representative exemplary embodiment, fine roughness may be provided by printing a fine pattern on the top surface of the nail sticker.

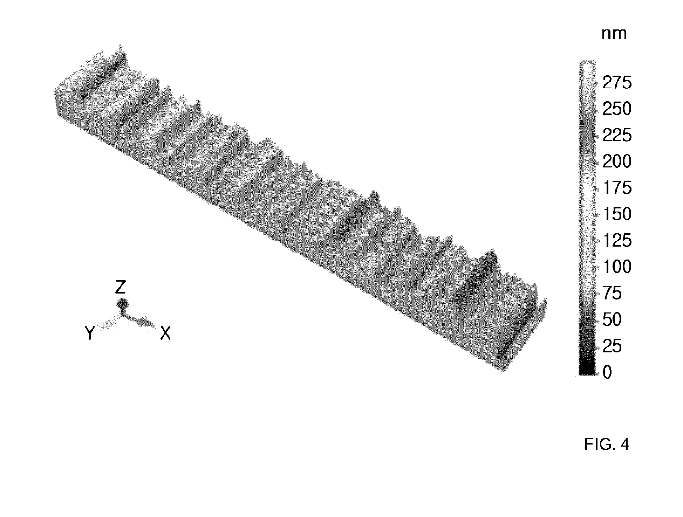

Specifically, by providing fine roughness by printing a fine pattern on the top surface of the nail sticker, as shown in FIG. 4, an embossing pattern may be formed on the nail sticker. As a result, adhesion between the nail sticker and the UV gel layer is enhanced because surface area and contact area with the UV gel layer are increased.

The printing may be performed by silk-screen printing, gravure printing, offset printing, or the like, but is not limited thereto. A regular pattern may be formed by the printing. However, if the same effect can be achieved by forming an irregular pattern by another method, it will be also included in the scope of the present disclosure.

Although the printing may be performed directly on the top surface of the nail sticker, a base primer layer may be formed on the nail sticker and then a print layer may be formed on the base primer layer through printing. Particularly, in the latter case, unlike when the surface roughness is provided physically or when the print layer is formed without the base primer layer, the solvent remaining without being completely dried in the print layer may partly swell the nail sticker, thereby enhancing adhesion of the UV gel layer and providing improved glossy effect of the UV gel layer.

Particularly, as when the roughness is provided by rolling, when the surface roughness satisfies 0.04a ≤ Rₐ ≤ 100a and 1.6z ≤ R_{z} ≤ 400z, the UV gel layer can be stably attached without interlayer delamination for at least 3 days on average.

In addition, when the surface roughness satisfies 1a ≤ Rₐ ≤ 50a and 5z ≤ R_{z} ≤ 200z, not only the adhesion between the nail sticker and the UV gel layer is ensured as descried above but also the problem that the end of the cuticle is peeled can be solved.

Particularly, when the surface roughness satisfies 5a ≤ Rₐ ≤ 10a and 10z ≤ R_{z} ≤ 100z, the problem that the opposite end of the cuticle is chipped can be further solved in addition to the enhancement of adhesion between the nail sticker and the UV gel layer and the prevention of peeling at the end of the cuticle. That is to say, since the fine wrinkles formed on the surface of the nail sticker by embossing firmly hold the UV gel layer when the surface roughness satisfies the above-described condition, the problem that the both ends of the UV gel layer are peeled can be completely prevented.

In another representative exemplary embodiment, a UV-cured coating layer may be formed on the nail sticker.

The UV-cured coating layer may be formed using a composition for forming a UV-cured coating layer including (i) a UV-curable oligomer and (ii) a UV initiator. The composition for forming a UV-cured coating layer may further include (iii) a multifunctional monomer and (iv) an additive such as a diluent.

The nail sticker for coating a UV gel layer exhibits superior adhesion with the UV gel layer and more improved decorating effect when a UV-curable coating layer is introduced onto its surface than when a thermally curable coating layer is introduced. This may be because the unreacted UV-curable oligomer and the UV initiator included in the UV-cured coating layer participate again in reaction during the formation of the UV gel layer.

The UV-curable oligomer refers to an oligomer with both ends substituted with UV-curable functional groups. The UV-curable functional group may be an acrylate group, a vinyl group, etc., but is not limited thereto. The oligomer may have a degree of polymerization of 3-1,000, specifically 5-500, more specifically 10-100.

Examples of the UV-curable oligomer include diacrylated urethane oligomer, diacrylate oligomer, diacrylated ethylene glycol oligomer, diacrylate vinyl alcohol oligomer, ester acrylate oligomer, epoxy acrylate oligomer, spirane acrylate oligomer, etc., but are not limited thereto. Specifically, diacrylated urethane oligomer, diacrylated ethylene glycol oligomer or both may be used among them.

As used herein, the term diacrylated urethane oligomer refers to a urethane oligomer with both ends linked to functional groups such as acrylate or methacrylate, as shown in the following chemical formula. However, the chemical formula is presented only as an example and the scope of the present disclosure is not limited to the degree of polymerization shown in the following chemical formula. Specific examples of the diacrylated urethane oligomer include caprolactone, diethylene glycol, isophorone diisocyanate, 2-hydroxyethyl acrylate polymer (CAS No. 72162-39-1), but are not limited thereto.

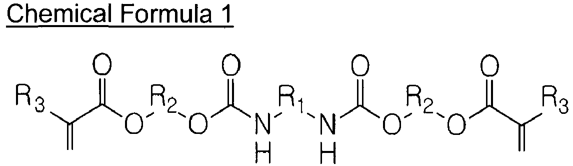

As used herein, the term diacrylated polyethylene glycol oligomer refers to a polymer (polyethylene glycol) or an oligomer of ethylene glycol with both ends linked to functional groups such as acrylate or methacrylate. Specifically, the diacrylated polyethylene glycol oligomer may be poly(ethylene glycol) 400 diacrylate, but is not limited thereto.

The UV initiator refers to an initiator capable of initiating polymerization upon UV radiation. The UV initiator may be specifically a ketone-based initiator, more specifically 1-hydroxycyclohexyl phenyl ketone, but is not limited thereto. The multifunctional monomer refers to a monomer having at least one polymerizable functional group. In particular, the multifunctional monomer may have two or more polymerizable functional groups or 1-3 polymerizable functional groups.

The monomer may include acrylate, diacrylate, triacrylate, etc., but is not limited thereto and various monomers may be used depending on the number of the functional groups. For example, as a monomer having one functional group, 2-hydroxyethyl acrylate (2-HEA), 2-hydroxyethyl methacrylate (2-HEMA), etc. may be used, without being limited thereto.

In an exemplary embodiment of the present disclosure, the composition for forming a UV-cured coating layer may have a viscosity measured at 25 °C using a Brookfield viscometer with spindle S31 of 5,000-1,000 and a tensile modulus measured with a UTM after curing of 25% or greater.

And, the composition for forming a UV-cured coating layer may either include a small amount of solvent or may be solvent-free. Specifically, the composition for forming a UV-cured coating layer may include 0-5 wt% of a solvent and 95-100 wt% of solid components.

In another exemplary embodiment of the present disclosure, the contents of (i) the UV-curable oligomer, (ii) the UV initiator and (iii) the multifunctional monomer may be controlled such that part of the UV-curable functional groups of the UV-curable oligomer or the multifunctional monomer remain uncured.

Particularly, it is very important to control the contents of the components of the UV-cured coating layer, in particular the UV initiator, such that 3-5% of the UV-curable functional groups included in the composition for forming a UV-cured coating layer remain without participating in reaction. If the flat nail sticker is applied on the curved natural nail, wrinkles may be formed on the nail sticker and the UV gel layer may partly come off the nail. This problem may be severe when the UV gel layer is directly coated on the transparent nail sticker. However, if the UV-curable functional groups of the above-described content remain uncured, they hold the nail sticker and prevent the above problem.

In another representative exemplary embodiment, a top primer layer may be formed on the nail sticker.

In an exemplary embodiment, the top primer layer may be formed by coating a composition for forming a top primer layer including a polymer for forming a top primer layer, a solvent, an additive, etc. on the nail sticker and evaporating the solvent, such that the polymer for forming a top primer layer forms a film. The polymer for forming a top primer layer may include polyurethane, polyacrylate, polyolefin, polyvinyl alcohol, polyvinyl chloride and blends or copolymers thereof. Specifically, polyurethane resin may be used.

In another exemplary embodiment, the composition for forming a top primer layer may have a surface tension measured by the dyne pen method of 28-72 dyne/cm, and the top primer layer may have a curing index measured as modulus after allowing to stand at 75 °C for 72 hours of 5-25 MPa.

In another exemplary embodiment, the composition for forming a top primer layer may include 36-42 wt% of polyurethane, 18-24 wt% of xylene and 35-41 wt% of cyclohexanone.

In another exemplary embodiment, the composition for forming a top primer layer may further include a UV-curable oligomer with both ends substituted with UV-curable functional groups and the content of the UV-curable oligomer may be 1-3% of the polymer for forming a top primer layer and 3-5% of the UV-curable functional groups included in the composition for forming a UV-cured coating layer. If the flat nail sticker is applied on the curved natural nail, wrinkles may be formed on the nail sticker and the UV gel layer may partly come off the nail. This problem may be severe when the UV gel layer is directly coated on the transparent nail sticker. By further including the UV-curable oligomer with the above-described content remain uncured, this problem can be prevented.

In another representative exemplary embodiment, a laminated film layer may be attached on the top surface of the nail sticker.

In an exemplary embodiment, the laminated film layer may be formed of a material having a tensile strength measured according to ASTM D638 of 5-200 MPa, an elongation measured according to ASTM D638 of 10-500%, a modulus in the elongation range of 0-10% measured according to ASTM D638 of 3-70 MPa, a surface tension measured by the dyne pen method of 28-72 dyne/cm, a curing index measured as modulus after allowing to stand at 75 °C for 72 hours of 10-50 MPa, a thickness measured by the dual gauge method of 2-50 µm, and a print adhesion measured according to the cross-cut tape test of ASTM D3359-02 of 2B or greater.

In the pencil test according to ASTM D3363, hardness is generally evaluated as 8 grades of 2B < B < HB < F < H < 2H < 3H < 4H. The hardness is smaller toward 2B and larger toward 4H. Unless otherwise specified, the pencil test according to ASTM D3363 means particularly the method described in EP 2417180 A1.

If the flat nail sticker is applied on the curved natural nail, wrinkles may be formed on the nail sticker and the UV gel layer may partly come off the nail. This problem may be severe when the UV gel layer is directly coated on the transparent nail sticker. If the laminated film layer has the above-described physical properties, it can firmly hold the nail sticker and prevent the above problem.

In another representative exemplary embodiment, the nail sticker may be formed of a material selected from polyvinyl chloride, polyacrylate, polystyrene, nylon, polyvinyl chloride, polyethylene, polypropylene, polyurethane, thermoplastic polyurethane (TPU), polyvinyl alcohol and blends thereof and may have two or more layers.

In an exemplary embodiment, a blend of a first resin selected from polyacrylate, polystyrene, nylon and a blend thereof and a second resin selected from polyethylene, polypropylene, polyurethane, thermoplastic polyurethane, polyvinyl alcohol and a blend thereof may be used. Through this, weak brittleness may be improved, excessively high rigidity may be reduced, and good print processability may be achieved.

In another exemplary embodiment, a second resin selected from polyethylene, polypropylene, polyurethane, thermoplastic polyurethane and a blend thereof may be laminated on a first resin selected from polyacrylate, polystyrene, nylon and a blend thereof. Through this, weak brittleness may be improved, excessively high rigidity may be reduced, and good print processability may be achieved.

In another exemplary embodiment, the nail sticker may be formed of a material having a tensile strength measured according to ASTM D638 of 5-200 MPa, an elongation measured according to ASTM D638 of 10-500%, a modulus in the elongation range of 0-10% measured according to ASTM D638 of 3-70 MPa, a surface tension measured by the dyne pen method of 28-72 dyne/cm, a curing index measured as modulus after allowing to stand at 75 °C for 72 hours of 10-50 MPa, a thickness measured by the dual gauge method of 20-180 µm, and a print adhesion measured according to the cross-cut tape test of ASTM D3359-02 of 2B or greater.

When coating the UV gel layer, it is important to match the edge portion with that of the natural nail. Otherwise, peeling may occur from the edge portion. In this case a customer cannot apply the UV gel layer to the natural nail himself/herself but has to buy service from a professional nail shop at high cost. However, if the nail sticker has the above-described physical properties, the peeling problem is avoided even when the edge portion is not precisely matched when coating the composition for forming a UV-cured coating layer on the nail sticker. Accordingly, the customer can perform nail care at low cost without having to visit the professional nail shop.

In accordance with the embodiments of the present disclosure, the need of soaking a nail in a harmful solution for a long time for removal of a UV gel may be avoided while maintaining the advantages of the UV gel.

Also, in accordance with the embodiments of the present disclosure, the problem that the opposite end of the cuticle is chipped may be solved while maintaining the advantages of the UV gel.

In addition, the problem that the end of the cuticle is peeled may be solved while maintaining the advantages of the UV gel.

While the present disclosure has been described with respect to the specific embodiments, it will be apparent to those skilled in the art that various changes and modifications may be made without departing from the spirit and scope of the disclosure as defined in the following claims.

## Claims

1. A nail sticker for coating a UV gel layer, wherein a top surface of the nail sticker is embossed.

2. The nail sticker for coating a UV gel layer according to claim 1, wherein said embossing is performed by passing the nail sticker for coating a UV gel layer between a first roll and a second roll and an embossing pattern is formed on at least one of the first roll and the second roll.

3. The nail sticker for coating a UV gel layer according to claim 1, wherein the nail sticker for coating a UV gel layer has a surface roughness of 5a ≤ Rₐ ≤ 10a and 10z ≤ R_{z} ≤ 100z as a result of said embossing.

4. A nail sticker for coating a UV gel layer, which has a print layer formed on a top surface of the nail sticker.

5. The nail sticker for coating a UV gel layer according to claim 4, wherein the print layer is formed by a method selected from silk-screen printing, gravure printing and offset printing and the print layer is formed on a base primer layer formed on a top surface of the nail sticker for coating a UV gel layer.

6. The nail sticker for coating a UV gel layer according to claim 4, wherein the nail sticker for coating a UV gel layer has a surface roughness of 5a ≤ Rₐ ≤ 10a and 10z ≤ R_{z} ≤ 100z.

7. A nail sticker for coating a UV gel layer, which has a UV-cured coating layer formed on a top surface of the nail sticker.

8. The nail sticker for coating a UV gel layer according to claim 7, wherein the UV-cured coating layer is formed by coating a composition for forming a UV-cured coating layer comprising a UV-curable oligomer and a UV initiator on the nail sticker for coating a UV gel layer and irradiating UV.

9. The nail sticker for coating a UV gel layer according to claim 8, wherein the composition for forming a UV-cured coating layer further comprises a multifunctional monomer.

10. The nail sticker for coating a UV gel layer according to claim 8, wherein the UV-curable oligomer is an oligomer with both ends substituted with UV-curable functional groups, the UV-curable functional group being selected from an acrylate group and a vinyl group, the oligomer is selected from a urethane oligomer, an acrylate oligomer, an olefin oligomer and a vinyl alcohol oligomer, and the oligomer has a degree of polymerization of 10-100.

11. The nail sticker for coating a UV gel layer according to claim 8, wherein part of the UV-curable functional groups included in the UV-cured coating layer remain unreacted after UV curing and the content of the unreacted UV-curable functional groups is 3-5% of the UV-curable functional groups included in the composition for forming a UV-cured coating layer.

12. A nail sticker for coating a UV gel layer, which has a top primer layer formed on a top surface of the nail sticker.

13. The nail sticker for coating a UV gel layer according to claim 12, wherein the top primer layer is formed by coating a composition for forming a top primer layer comprising a polymer for forming a top primer layer, a solvent and an additive on the nail sticker and evaporating the solvent partly or completely.

14. The nail sticker for coating a UV gel layer according to claim 13, wherein the polymer for forming a top primer layer is selected from polyurethane, polyacrylate, polyolefin, polyvinyl alcohol and polyvinyl chloride, the composition for forming a top primer layer has a surface tension measured by the dyne pen method of 28-72 dyne/cm, and the top primer layer has a curing index measured as modulus after allowing to stand at 75 °C for 72 hours of 5-25 MPa.

15. The nail sticker for coating a UV gel layer according to claim 13, wherein the composition for forming a top primer layer further comprises a UV-curable oligomer with both ends substituted with UV-curable functional groups and the content of the UV-curable oligomer is 1-3% of the polymer for forming a top primer layer and 3-5% of the UV-curable functional groups included in the composition for forming a UV-cured coating layer.

16. A nail sticker for coating a UV gel layer, which has a laminated film layer attached on a top surface of the nail sticker.

17. The nail sticker for coating a UV gel layer according to claim 16, wherein the laminated film layer has a tensile strength measured according to ASTM D638 of 5-200 MPa, an elongation measured according to ASTM D638 of 10-500%, a modulus in the elongation range of 0-10% measured according to ASTM D638 of 3-70 MPa, a surface tension measured by the dyne pen method of 28-72 dyne/cm, a curing index measured as modulus after allowing to stand at 75 °C for 72 hours of 10-50 MPa, a thickness measured by the dual gauge method of 2-50 µm, and a print adhesion measured according to the cross-cut tape test of ASTM D3359-02 of 2B or greater.

18. A UV gel layer-coated nail sticker, which has a UV gel layer formed on a nail sticker.

19. The UV gel layer-coated nail sticker according to claim 18, wherein the nail sticker is the nail sticker for coating a UV gel layer according to any one of claims 1 to 15.

20. A nail sticker kit for coating a UV gel layer comprising a nail sticker and a composition for forming a UV-cured coating layer.

21. The nail sticker kit for coating a UV gel layer according to claim 20, wherein the nail sticker is the nail sticker for coating a UV gel layer according to any one of claims 1 to 15.

22. The nail sticker kit for coating a UV gel layer according to claim 20, wherein the composition for forming a UV-cured coating layer comprises a composition for forming a UV base gel layer and a composition for forming a UV top gel layer.

23. The nail sticker kit for coating a UV gel layer according to claim 20, wherein the nail sticker kit for coating a UV gel layer further comprises a UV light source providing means.

24. The nail sticker kit for coating a UV gel layer according to claim 20, wherein the nail sticker kit for coating a UV gel layer further comprises a coating aiding means for coating the composition for forming a UV top gel layer.

25. A nail care method using a UV gel layer-coated nail sticker, comprising:
attaching a nail sticker on a natural nail;
coating a composition for forming a UV base gel layer on the nail sticker;
forming a UV base gel layer by exposing the coated composition for forming a UV base gel layer to UV light;
coating a composition for forming a UV top gel layer on the UV base gel layer; and
forming a UV top gel layer by exposing the coated composition for forming a UV top gel layer to UV light.

26. The nail care method using a UV gel layer-coated nail sticker according to claim 25, wherein the nail sticker is the nail sticker for coating a UV gel layer according to claim 1.

27. The nail care method using a UV gel layer-coated nail sticker according to claim 25, wherein the nail sticker is the nail sticker for coating a UV gel layer according to claim 4.

28. The nail care method using a UV gel layer-coated nail sticker according to claim 25, wherein the nail sticker is the nail sticker for coating a UV gel layer according to claim 7.

29. The nail care method using a UV gel layer-coated nail sticker according to claim 25, wherein the nail sticker is the nail sticker for coating a UV gel layer according to claim 12.

30. The nail care method using a UV gel layer-coated nail sticker according to claim 25, wherein the nail sticker is the nail sticker for coating a UV gel layer according to claim 16.
